# EUROPEAN PATENT APPLICATION

(11) **EP 1 238 671 A1**
(43) Date of publication of application: **11.09.2002**
(21) Application number: 01200815.7
(22) Date of filing: 05.03.2001
(51) Int. Cl.: A61K 35/14, A61K 35/44, A61K 35/50, A61J 1/05

(54) **Cord blood banks and uses thereof**

(71) Applicant: Stichting Eurocord Nederland, 2333 ZA Leiden (NL)
(72) Inventor: The designation of the inventor has not yet been filed
(74) Representative: Prins, Adrianus Willem

(57) **Abstract**

Provided is a bank of cord blood cells from which cells may be withdrawn from storage for both autologous and allogeneic purposes. Preferably, cord blood cells from a single individual are stored in such a way that they can be easily be used to prepare both an autologous and an allogeneic graft. The invention further provides means and methods for generating and using a combined autologous and allogeneic cord blood bank.

## Description

The invention relates to the field of medicine. The invention in particular relates to the field of transplantation medicine.

Transplantation of hemopoietic cells has become a standard treatment for a wide variety of diseases. Many different genetic diseases affecting the hemopoietic system can be cured by successful hemopoietic stem cell transplantation. Some of the negative effects of cancer treatments on the blood cell system of the patient can be ameliorated by infusion of hemopoietic stem cells.

Although hemopoietic stem cell transplantation, as a treatment of disease, is very successful, there are also risks associated with it. It is important, as for any transplantation, that the immune system of the recipient does not reject the grafted cells. When rejection of the grafted cells by the immune system of the recipient occurs, so-called host versus graft(HvG) disease, the positive effects of the transplantation disappear. On the other hand, since blood cells form the basis of the immune system it is also possible that the grafted donor cells mount an immune response against the recipient. This so-called graft versus host (GvH) disease can become very severe and is an important risk factor in the overall mortality in stem cell transplantation. The risk of HvG and GvH disease is particularly a problem in allogeneic transplantation settings where the donor cells are derived from a genetically different individual. Not all differences in the genetic make up of individuals form an equal risk in the development of HvG or GvH. Differences in so-called major histocompatibility antigens are more prone to elicit such responses in the recipient than differences in so-called minor histocompatibility antigens. To reduce rejection problems, donors and patients can be matched for these and other antigens. However, considering the plurality of differences that can occur, and that are known to affect grafting of cells, it is difficult to find donors that are completely matched for all major and minor histocompatibility antigens. Suitable donors are much more frequently found among siblings than among unrelated individuals. However, often a patient does not have a sibling that is suitable as a donor. For these patients, suitable donors are selected from normal individuals which are typically matched for the major histocompatibility antigens. To facilitate the search for suitable donors so-called stem cell banks are set up. These banks can consist of stem cells collected from normal individuals that are stored under conditions allowing long term survival of the stored cells. Together with information on histocompatibility antigens and preferably other important transplantation information, such banks can provide a good source of stem cells grafts. However, stem cell banks need to be very large to allow for a reasonable chance of obtaining a suitable graft for a given patient. This is largely due to the fact that there are many different histocompatibility antigens, and that individuals may carry combinations of histocompatibility antigens. The requirement of very large banks has prompted initiatives to implement virtual stem cell banks. Individuals willing to donate stem cells are typed for histocompatibility antigens and possibly other important transplantation information. This information is stored in a database. An individual in need for a transplant is typed and the results of the typing are compared with the information in the database. In case a match is found, the potential donor is contacted and asked to donate some stem cells. In this way very large virtual banks can be generated. However, also these virtual banks do not succeed in improving the chances very much for finding a suitable unrelated donor for any given patient. The number of combinations of major and also minor histocompatibility antigens is just too large.

A relatively new development in the field of stem cell banks is the generation of banks comprising cord blood. Cord blood is typically collected at birth of a child and typically contains sufficient stem cells to perform a transplant in at least children. Cord blood transplantation technology is still improving. Even adults can be successfully transplanted with cord blood cells, moreover, there are strong indications that the young and immunologically naïve cord blood cells are less prone to induce GvH.

An attractive feature of cord blood cells is that they can be collected without burdening the donor. The cord blood is usually discarded upon delivery and separation of the cord from the baby. In addition, the cord blood is collected at a time point in life wherein the individual is typically healthy and not the host of infectious agents.

The availability of easily collectable grafts that are suitable for transplantation purposes has initiated the generation of cord blood banks. Parents are particularly interested in cord blood bank facilities. They want to provide the newly born with a possibility to obtain a suitable graft, when the baby is in need for a graft later in life. These banks are called autologous cord blood banks. Only the original donor is entitled to withdraw the cord blood from storage to prepare a graft for him or herself. Considering that many of these individuals will never be in need for a stem cell graft it is anticipated that many of the cord bloods will never be withdrawn from autologous banks.

Hospitals and midwives therefore ask parents of newly borns for permission to collect the cord blood of the baby for the generation of an allogeneic cord blood bank, for unrelated stem cell transplantation. Considering that many individuals are in need of a suitable stem cell graft, it is anticipated that blood in stored allogeneic banks will be withdrawn more frequently than blood stored in autologous banks. Allogeneic banks are therefore clinically much more relevant.

The generation of allogeneic cord blood banks, though clinically much more relevant, is a much more difficult task. With the growing awareness of the uses of cord blood, parents are typically hesitant to donate cord blood of their newborn to help other people. Since with that act they donate an ideal graft for their own child. In one aspect the invention provides a solution to this problem. In this aspect the invention provides a blood bank comprising cord blood cells for use in autologous as well as allogeneic transplantation. In this way parents can donate the cord blood and be reasonably certain that the cord blood is still available for use in the event that the child is in need of it.The cells in the bank can be used for a large variety of different autologous and allogeneic purposes. One these is of course the transplantation of hemopoietic stem cells. However, cord blood also contains many other types of cells such as the so-called mesenchymal stem cell (see also references 1-4). The mesenchymal stem cell are multipotent and serve as long-lasting precursors for bone marrow stromal cells, bone cartilage, muscle and connective tissue. Transplantation of mesenchymal stem cells can therefore be useful for the (re)generation of stroma, cartilage, muscle etc. Transplantation of stored cells does not have to be done immediately after recovery of the cells from storage. Stored cells may be cultured and/or expanded before transplantation. Cord blood cells may, of course, also be cultured and/or expanded prior to incorporation into the bank. Cord blood cells may also be fractionated before or after storage. If not all of the fractions are utilized the act of fractionation may result in some types of cells to be under or over represented in the stored cells, or in the cells used in the transplant. Considering the pluri- and multipotent nature of some types of cord blood cells, it is anticipated that stored cells can be used for many different transplantation purposes. Hemopoietic stem cell transplantation is one of them, the *in vitro* culture of cord blood cells which are subsequently transplanted is another. Another non-limiting example is the *in vitro* the generation of human tissue that is subsequently transplanted. This is largely due to the fact that with the current typing systems very large banks are needed to find perfect matches. Thus the chance that an individual is in need of a particular cord blood is relatively low. This aspect of the invention allows the majority of the stored cord bloods to be available for future use by the donating child. The number of cases where a donating child, later in life, will not be able to obtain its cord blood cells from the bank will be relatively low, particularly in view of the fact that only a minority of the donating children will ever be in need of a transplant. However, although the chances are good, it is not certain that a donor will be able to obtain the donated cells later in life. In a preferred embodiment of the invention cord blood cells collected from cord blood of a single individual are used for the preparation of an autologous and an allogeneic transplant. In this way not all cells are handed out for a transplant but at least some cord blood cells collected from said individual remain in storage. In this embodiment, a donor will always have access to the stored cells when the donor is in need of the cells for the first time. Of course in case the donor requires more than one transplant of hemopoietic cells, the bank might not have (sufficient) cells in storage to provide for more than one autologous transplant. To facilitate the double use of a single cord blood it is preferred that said cord blood cells collected from cord blood of a single individual are stored in two or more containers. In this case one container can be marked for autologous transplantation and another container can be marked for allogeneic transplantation. Preferably, said containers are physically linked through a separable linkage. In this way containers may be filed and stored in a single pass. This is advantageous in, for instance, quality control.

A bank of the invention comprising cord blood collected from a single individual for use in allogeneic and autologous transplantation can of course further comprise cord blood cells collected from the cord blood of a single individual for use in only an allogeneic or only an autologous transplantation.

In another aspect the invention provides a method for handing out cord blood cells from a cord blood bank comprising, handing out a first sample of cord blood cells for an allogeneic application and, handing out second sample of cord blood cells for an autologous application. Preferably said first and said second sample comprise cord blood cells collected from a single individual.

In yet another aspect the invention provides a container for storing cord blood cells for use in a cord blood bank according to the invention. Preferably, said container further comprises cord blood cells.

In still another aspect the invention provides a collection of containers linked to each other through a separable linkage for use in a cord blood bank or a method of the invention. Preferably, said collection comprises cord blood cells collected from a single individual.

### REFERENCES

1. Erices, A *et al*. British J. of Haematol. 2000, **109:** 235-242.
2. Huss, R *et al*. Stem Cells. 2000, **18:**252-260.
3. Almeida-Porada, G. *et al*. Exp. Hematology. 1999, **27:** 1569-1575.
4. Devine, S.M. and Hoffman, R. 2000, 7:358-363

## Claims

1. A cord blood bank comprising cord blood cells for use in an autologous and an allogeneic application.

2. A cord blood bank according to claim 1 comprising cord blood cells for use in autologous and allogeneic transplantation.

3. A cord blood bank according to claim 1 or claim 2, wherein cord blood cells collected from cord blood of a single individual are used for the preparation of an autologous and an allogeneic transplant.

4. A cord blood bank according to anyone of claims 1-3, wherein cord blood cells collected from cord blood of a single individual are stored in two or more containers.

5. A cord blood bank according to claim 4, wherein said containers are physically linked through a separable linkage.

6. A method for handing out cord blood cells from a cord blood bank comprising
■ handing out a first sample of cord blood cells for an allogeneic transplant and
■ handing out second sample of cord blood cells for an autologous transplant.

7. A method according to claim 6, wherein said first and said second sample comprise cord blood cells collected from a single individual.

8. A container for storing cord blood cells for use in a cord blood bank according to any one of claims 1-5.

9. A container according to claim 8, further comprising cord blood cells.

10. A collection of containers according to claim 8 or claim 9 linked to each other through a separable linkage.

11. A collection of containers according to claim 10, comprising cord blood cells collected from cord blood of a single individual.
